Europäisches Patentamt

European Patent Office

Office européen des brevets

(11) Veröffentlichungsnummer: **0 248 400**
**A2**

(12) **EUROPÄISCHE PATENTANMELDUNG**

(21) Anmeldenummer: **87107960.4**

(22) Anmeldetag: **02.06.87**

(51) Int. Cl.⁴: **C12N 9/04 , C12P 19/02**

Der (Die) Mikroorganismus (Mikroorganismen) ist (sind) bei FRI unter der (den) Nummer(n) FERM BP - 1267, FERM BP - 1265, FERM BP - 1266, FERM BP - 1268 hinterlegt worden.

(30) Priorität: **03.06.86 GB 8613430**
        **14.04.87 GB 8708907**

(43) Veröffentlichungstag der Anmeldung:
**09.12.87 Patentblatt 87/50**

(84) Benannte Vertragsstaaten:
**AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Fujiwara, Akiko**
**Fueta 1059-7 Kamakura-shi**
**Kanagawa-ken(JP)**
Erfinder: **Hoshino, Tatsuo**
**Fueta 808-47 Kamakura-shi**
**Kanagawa-ken(JP)**
Erfinder: **Sugisawa, Teruhide**
**Kohnandai 8-26-10 Kohnan-ku**
**Yokohama-shi Kanagawa-ken(JP)**

(74) Vertreter: **Wuesthoff, Franz, Dr.-Ing. et al Patentanwälte Wuesthoff -v. Pechmann-Behrens-Goetz Schweigerstrasse 2 D-8000 München 90(DE)**

(54) **Enzym und Verfahren zu seiner Herstellung.**

(57) Die erfindungsgemässe neue L-Sorbose-Dehydrogenase katalysiert die Oxidation von L-Sorbose zu L-Sorboson, dem Vorläufer der 2-Keto-L-gulonsäure, die ihrerseits ein wichtiges Zwischenprodukt bei der Herstellung von Vitamin C ist.

Die erfindungsgemässe Herstellung der neuen L-Sorbose-Dehydrogenase erfolgt durch Kultivieren eines zur Gattung Gluconobacter gehörenden Mikroorganismus oder einer Mutante davon, die zur Herstellung der neuen L-Sorbose-Dehydrogenase in Zellmaterial befähigt sind, Zertrümmern des Zellmaterials, Isolieren der L-Sorbose-Dehydrogenase aus zellfreiem Extrakt des zertrümmerten Zellmaterials, vorzugsweise aus der Membranfraktion des Zellmaterials, sowie Reinigen der L-Sorbose-Dehydrogenase.

EP 0 248 400 A2

## Enzym und Verfahren zu seiner Herstellung

Die vorliegendende Erfindung betrifft die neue Coenzym-unabhängige L-Sorbose-Dehydrogenase sowie ein Verfahren zu deren Herstellung.

Die erfindungsgemässe L-Sorbose-Dehydrogenase katalysiert die Oxidation von L-Sorbose zu L-Sorboson, dem Vorläufer für 2-Keto-L-gulonsäure, die ein wichtiges Zwischenprodukt bei der Herstellung von Vitamin C ist.

Eine Reaktion zur mikrobiellen Umwandlung von L-Sorbose in L-Sorboson ist bekannt. Die Herstellung von L-Sorboson aus L-Sorbose unter Verwendung von zellfreien Extrakten von Mikroorganismen ist aus verschiedenen Publikationen bekannt geworden. Gemäss der US-Patentschrift Nr. 3.912.592 ermöglichen die zu den Gattungen Gluconobacter, Pseudomonas, Acinetobacter, Bacillus, Sarcina, Streptomyces, Serratia, Aerobacter, Mycobacterium und Paecilomyces gehörenden Mikroorganismen eine solche Umwandlung. Makover et al. [Biotechnol. Bioeng. 17, 1485-1514 (1975)] berichten erstmalig über die Charakterisierung des Enzyms von Pseudomonas putida ATCC 21812, jedoch wird die Isolierung dieses Enzyms dort nicht beschrieben. Kitamura et al. [Biotechnol. Bioeng. 17 , 349-359 (1975)] berichten, dass die Aktivität der in Gluconobacter melanogenus IFO 3293 befindlichen L-Sorbose-Dehydrogenase durch Phenazinmethosulfat, Methylenblau oder Kaliumferricyanid als Elektronenakzeptor stimuliert werde. Allerdings konnten sie das Enzym nicht isolieren.

Wie oben gesagt, verlautet bisher nichts über ein gereinigtes Enzym, das die Aktivität zur Oxidation von L-Sorbose zu L-Sorboson aufweist.

Es wurde nun gefunden, dass das aus der Membranfraktion von Zellen bestimmter Mikroorganismen isolierte, gereinigte Enzym die Oxidation von L-Sorbose zu L-Sorboson spezifisch katalysiert. Die vorliegende Erfindung beruht auf diesem Befund.

Gegenstand der vorliegenden Erfindung ist die neue L-Sorbose-Dehydrogenase, die die Oxidation von L-Sorbose zu L-Sorboson katalysiert und eine hohe Substratspezifität aufweist. Ein weiterer Gegenstand ist ein Verfahren zur Herstellung der neuen L-Sorbose-Dehydrogenase durch Kultivieren eines zur Gattung Gluconobacter gehörenden Mikroorganismus oder einer Mutante davon, die die Herstellung der neuen L-Sorbose-Dehydrogenase in Zellmaterial ermöglichen, Zertrümmern des Zellmaterials, Isolieren der L-Sorbose-Dehydrogenase aus zellfreiem Extrakt des zertrümmerten Zellmaterials, vorzugsweise aus der Membranfraktion des Zellmaterials, sowie Reinigen der L-Sorbose-Dehydrogenase.

Die physiko-chemischen Eigneschaften der gereinigten neuen L-Sorbose-Dehydrogenase, deren Herstellung in den nachfolgenden Beispielen veranschaulicht wird, sind wie folgt:

(1) Enzymaktivität

Die L-Sorbose-Dehydrogenase der vorliegenden Erfindung katalysiert die Oxidation von L-Sorbose zu L-Sorboson in Gegenwart eines Elektronenakzeptors gemäss der folgenden Reaktionsgleichung:

$$\text{L-Sorbose + Elektronenakzeptor}$$
$$\downarrow$$
$$\text{L-Sorboson + reduzierter Elektronenakzeptor}$$

Das Enzym benützt Sauerstoff nicht als Akzeptor, sondern 2,6-Dichlorphenolindophenol (im folgenden als DCIP bezeichnet), Phenazinmethosulfat, Wurster's Blue, Ferricyanid, Coenzym Q und Cytochrom C können als Elektronenakzeptoren verwendet werden.

Zur Bestimmung der Enzymaktivität wurde die Absorptionsabnahme von DCIP bei 25°C und 600 nm spektrophotometrisch gemessen. Unter der Einheit der Enzymaktivität ist diejenige Menge Enzym, die die Reduktion von 1 μMol von DCIP pro Minute katalysiert, zu verstehen. Der Extinktionscoeffizient von DCIP bei einem pH-Wert von 7,0 ist 14,5 mM$^{-1}$. Das Basis-Reaktionsgemisch wird unten angegeben. Das Gemisch wurde jeweils vor dem Test vorbereitet.

Basis-Reaktionsgemisch:

    0,1 M Kaliumphosphat

    Puffer (pH-Wert 7,0) enthaltend 0,3% Triton X-100       3     ml

    2,5 mM DCIP          0,45 ml

    Wasser              4,95 ml

Eine Küvette mit einer Schichtlänge von 1 cm im Lichtweg enthielt 0,4 ml des Basis-Reaktionsgemisches, 0,1 ml 1 M L-Sorbose, Enzymlösung und Wasser mit einem Endvolumen von 0,51 ml. Eine Referenzküvette enthielt sämtliche Komponenten mit der Ausnahme des Substrats. Die Reaktion wurde durch Zugabe des Substrats in Gang gebracht. Die Enzymaktivität bestimmte sich durch die anfängliche Reduktionsgeschwindigkeit von DCIP.

(2) <u>Substratspezifität</u>

Die Substratspezifität des Enzyms wurde unter Verwendung einer Standardenzymtestmethode bestimmt. Diese Methode unterscheidet sich von der unter (1) beschriebenen Methode dadurch, dass je 0,1 ml der verschiedenen Substratlösungen (1 M) anstelle von L-Sorbose verwendet wurde. Die Ergebnisse der Bestimung sind in der Tabelle 1 aufgeführt. Es stellte sich heraus, dass das erfindungsgemässe Enzym für die L-Sorbose ausgesprochen spezifisch ist.

## Tabelle 1

| Substrat | Relative Enzymaktivität (%) |
|---|---|
| Sorbose | 100 |
| Glucose | 0 |
| Mannit | 0 |
| Sorbit | 0 |
| Fructose | 0 |
| Glycerin | 0 |
| Adonit | 0 |
| Erythrit | 0 |
| Natriumgluconat | 0 |
| Calciumidonat | 0 |
| Mannose | 0 |
| Maltose | 0 |
| Xylose | 0 |
| Aethanol | 0 |
| Lactose | 0 |
| Sucrose | 0 |
| Galactose | 0 |
| Cellobiose | 0 |
| Inosit | 0 |
| Arabinose | 0 |

(3) Optimaler pH-Wert

Das Verhältnis zwischen der Reaktionsgeschwindigkeit der L-Sorbose-Dehydrogenase und dem pH-Wert wurde in McIlvaine-Puffer (Gemisch von 0,1 M Zitronensäure und 0,2 M Dinatriumphosphat) verschiedener pH-Werte bestimmt. Die Ergebnisse sind in der Tabelle 2 aufgeführt.

## Tabelle 2

| pH-Wert | Relative Aktivität (%) |
| --- | --- |
| 4,0 | 0 |
| 4,5 | 0 |
| 5,1 | 9,4 |
| 5,6 | 35,3 |
| 6,1 | 68,8 |
| 6,6 | 90,0 |
| 7,2 | 100 |
| 7,7 | 95,0 |
| 8,1 | 85,3 |

Das Enzym wies die grösste Enzymaktivität im pH-Bereich 6,5 bis 8,0 auf.

(4) Stabilität bei verschiedenen pH-Werten

Das gereinigte Enzym wurde zu McIlvaine-Puffer (Gemisch von 0,1 M Zitronensäure und 0,2 M Dinatriumphosphat) verschiedener pH-Werte gegeben, und das jeweilige Gemisch wurde 121 Stunden bei 4°C stehengelassen. Die Restaktivität wurde unter den Standardtestbedingungen, wie diese oben unter (1) beschrieben sind, bestimmt. Die Ergebnisse der Bestimmung sind in der Tabelle 3 aufgeführt.

## Tabelle 3

| pH-Wert | Relative Aktivität (%) |
| --- | --- |
| 4,0 | 0 |
| 4,5 | 0 |
| 5,0 | 0 |
| 5,5 | 0 |
| 6,0 | 23,8 |
| 6,5 | 60,0 |
| 7,0 | 100 |
| 7,5 | 75,9 |
| 8,0 | 57,5 |

Das gereinigte Enzym war zwischen den pH-Werten 6,5 und 8,0 relativ stabil.

(5) Wärmestabilität

Das gereinigte Enzym wurde 5 Minuten bei verschiedenen Temperaturen in 10 mM Kaliumphosphatpuffer (pH-Wert 7,0) inkubiert und danach in Eis/Wasser abgekühlt. Die Restaktivität wurde unter den Standardtestbedingungen, wie diese oben unter (1) beschrieben sind, gemessen. Die Ergebnisse sind in der Tabelle 4 aufgeführt.

## Tabelle 4

| Temperatur (°C) | Relative Aktivität (%) |
|---|---|
| 0 | 100 |
| 22 | 100 |
| 25 | 100 |
| 30 | 97,9 |
| 36 | 86,6 |
| 41 | 69,2 |
| 46 | 47,5 |
| 55 | 14,7 |
| 65 | 10,3 |

Das gereinigte Enzym war bis zu 30°C stabil, verlor jedoch nach Inkubation bei 46°C ca. 50% bzw. 90% seiner Aktivität .

(6) Abhängigkeit der Aktivität von der Temperatur

Die Enzymaktivität von L-Sorbose-Dehydrogenase wurde bei Temperaturen von 20°C bis 58°C unter den Standardtestbedingungen, wie diese oben unter (1) beschrieben sind, bestimmt. Die Ergebnisse sind in der Tabelle 5 aufgeführt.

## Tabelle 5

| Temperatur (°C) | Relative Aktivität (%) |
|---|---|
| 20 | 28,2 |
| 25 | 26,7 |
| 29 | 35,9 |
| 36 | 50,8 |
| 40 | 70,7 |
| 45 | 93,8 |
| 48 | 100 |
| 55 | 91,8 |
| 58 | 77,0 |

Die Aktivität des erfindungsgemässen Enzyms nimmt bei steigender Temperatur bis auf 48°C zu.

### (7) Molekulargewicht

Die Enzymlösung wurde auf eine Sephadex G-200-Säule (1,0 × 100 cm), die mit 10 mM Kaliumphosphatpuffer (pH-Wert 7,0) enthaltend 0,3% Triton X-100 und 0,2 M L-Sorbose äquilibriert worden war, aufgetragen und mit demselben Puffer eluiert.

Das Molekulargewicht des Enzyms betrug 210.000 ± 20.000 (durch Gelfiltration bestimmt). Da allerdings das erfindungsgemässe Enzym ein Membranprotein ist und von der Membran unter Verwendung eines oberflächenaktiven Mittels solubilisiert wird, entspricht das berechnete Molekulargewicht nicht unbedingt dem eigentlichen Molekulargewicht des Enzymproteins. Das Enzym zeigte durch Polyacrylamidgel-Elektrophorese in Gegenwart von Natriumdodecylsulfat (SDS) eine einzelne Bande, die einem Molekulargewicht von ca. 58.000 ± 5.000 entsprach, was etabliert, dass das erfindungsgemässe Enzym aus homologen Untereinheiten besteht.

### (8) Bestimmung des Km-Wertes

Die Geschwindigkeit der Oxidation bei variierender Konzentration von L-Sorbose (0,5 mM bis 320 mM) wurde unter Verwendung der oben unter (2) beschriebenen Methode gemessen, um die Km-Werte für L-Sorbose festzustellen. Es wurde gefunden, dass die maximale Reaktionsgeschwindigkeit bei einer Substratkonzentration von ca. 200 mM erreicht wurde. Für die scheinbare Michaelis-Konstante (Km) wurde unter Verwendung von DCIP als Elektronenakzeptor ein Wert von 102 ± 10 mM berechnet.

### (9) Einfluss von Metallionen

Unter Verwendung der oben unter (1) beschriebenen Testmethode wurde der Einfluss von verschiedenen Metallionen auf die Enzymaktivität untersucht. Die Ergebnisse der Bestimmung sind in der Tabelle 6 aufgeführt. $Co^{2+}$ und $Fe^{3+}$ wirkten stimulierend und $Cu^{2+}$ hemmend.

Tabelle 6

| Metall | Konzentration (mM) | Relative Aktivität (%) |
|---|---|---|
| $CaCl_2$ | 0,4 | 100 |
| | 0,8 | 100 |
| $CoCl_2 \cdot 6H_2O$ | 0,4 | 109,2 |
| | 0,8 | 118,4 |
| $CuSO_4$ | 0,8 | 0 |
| $CuSO_4 \cdot 5H_2O$ | 0,08 | 30,9 |
| $CuCl_2 \cdot 2H_2O$ | 0,4 | 0 |
| $Cu(NO_3)_2 \cdot H_2O$ | 0,3 | 2,27 |
| $Fe(SO_4)_3 \cdot xH_2O$ | 0,4 | 121,3 |
| | 0,9 | 106,4 |
| $MgSO_4 \cdot 7H_2O$ | 0,4 | 100 |
| | 0,8 | 100 |
| $MgCl_2 \cdot 6H_2O$ | 0,8 | 83,3 |
| $MnCl_2 \cdot 4H_2O$ | 0,4 | 100 |
| | 0,9 | 98,3 |
| $MnSO_4 \cdot H_2O$ | 0,4 | 100 |
| $Na_2MoO_4 \cdot 2H_2O$ | 0,3 | 86,1 |
| | 1,06 | 69,6 |
| TiCl | 0,4 | 100 |
| | 0,8 | 100 |
| | 1,8 | 100 |
| $ZnSO_4 \cdot 7H_2O$ | 0,4 | 100 |
| | 0,9 | 100 |
| $NiSO_4 \cdot 6H_2O$ | 0,4 | 100 |
| | 0,9 | 100 |
| ohne | – | 100 |

(10) Einfluss von Hemmstoffen

Unter Verwendung der oben unter (1) beschriebenen Testmethode wurde der Einfluss verschiedener Hemmstoffe auf die Enzymaktivität untersucht. Die Ergebnisse sind in der Tabelle 7 aufgeführt. Chinin, Monojodacetat, Natriumfluoracetat und Natriumfluorid hemmten teilweise die Enzymaktivität.

### Tabelle 7

| Hemmstoff | Konzentration (mM) | Hemmung (%) |
|---|---|---|
| AeDTA | 0,94 | 0 |
|  | 5,2 | 13,3 |
| Chinin | 1,85 | 0 |
|  | 4,24 | 30,5 |
| N-Aethyl- | 1,85 | 0 |
| maleimid | 4,24 | 0 |
| Natriumazid | 1,85 | 0 |
|  | 4,24 | 0 |
| Monojod- | 1,85 | 0 |
| acetat | 4,24 | 34,6 |
|  | 9,37 | 68,5 |
| PCMB (p-Chlor- | 0,02 | 0 |
| mercuribenzoat) | 0,04 | 0 |
|  | 0,17 | 0 |
| $Na_2HAs \cdot O_4 \cdot 7H_2O$ | 1,85 | 0 |
|  | 4,24 | 0 |
| Natriumfluor- | 1,85 | 0 |
| acetat | 4,24 | 8,6 |
|  | 9,37 | 26,5 |
| Natriumfluorid | 1,85 | 10,3 |
|  | 4,24 | 10,3 |
|  | 9,37 | 22,1 |
| Kaliumcyanid | 0,88 | 0 |

(11) Reinigung

Die Reinigung von L-Sorbose-Dehydrogenase kann mittels bekannten Reinigungsmethoden bzw. durch Kombination solcher bekannter Reinigungsmethoden durchgeführt werden, z.B. Ionenaustauscherchromatographie, Flüssigkeitschromatographie, Gelfiltration, Gelelektrophorese, Aussalzen und Dialyse.

Die erfindungsgemässe L-Sorbose-Dehydrogenase kann durch Kultivieren eines entsprechenden Mikroorganismus, Zertrümmern der Zellen und Isolieren und Reinigen der L-Sorbose-Dehydrogenase aus zellfreiem Extrakt des zertrümmerten Zellmaterials, vorzugsweise aus der Membranfraktion des Zellmaterials, hergestellt werden.

Die in der vorliegenden Erfindung verwendeten Mikroorganismen gehören der Gattung Gluconobacter oder Mutanten davon an. Nach der neuesten Klassifikation fallen sämtliche Gluconobacter-Stämme unter die Spezies Gluconobacter oxydans. Die morphologischen und physiologischen Kennzeichen der Stämme, die Gluconobacter oxydans angehören, sind in "Bergey's Manual of Systematic Bacteriology", Vol. I, 275-278 (1984) und F. Gossele et al., International J. System. Bacteriol. Vol. 33, 65-81 (1983) beschrieben.

Die der Gattung Gluconobacter angehörenden Mikroorganismen, die im Rahmen der vorliegenden Erfindung verwendet werden, können aus natürlichen Quellen isoliert werden bzw. sind bei Kultursammlungen erhältlich. Auch derivierte Mutanten können gemäss der vorliegenden Erfindung verwendet werden.

Die in der vorliegenden Erfindung verwendeten Mutanten können erhalten werden durch Behandlung eines Wildtyp-Stammes mit einem Mutagen, wie UV-Bestrahlung, Röntgenbestrahlung, γ-Bestrahlung oder Behandlung mit salpetriger Säure oder anderen geeigneten Mutagenen, oder durch Isolierung eines Klons, der durch spontane Mutation auftritt. Diese Mutationen eines Wildtyp-Stammes oder eines Mutantenstammes davon können auf an sich bekannte Weise durchgeführt werden. Viele dieser Methoden sind in der Literatur beschrieben, z.B. "Chemical Mutagens", Herausgeber Y. Tajima, T. Yoshida und T. Kada, Kodansha Scientific Inc., Tokyo, Japan, 1973.

Die erfindungsgemässen Mutanten sind auch durch Fusion der zur Spezies Gluconobacter oxydans gehörenden Stämme und durch Kombination von Mutagenese und/oder Fusion zugänglich.

Beispiele von Stämmen, die bevorzugt verwendet werden, sind Gluconobacter oxydans UV-10, Gluconobacter oxydans E-1, Gluconobacter oxydans H-2 und Gluconobacter oxydans L-8. Diese Mikroorganismen sind bei der Agency of Industrial Science and Technology, Fermentation Research Institute, Japan unter den folgenden Hinterlegungsnummern deponiert:

|  |  |  | Zugriffs-Nr. |
|---|---|---|---|
| Gluconobacter oxydans UV-10 | FERM-P Nr. 8422, | FERM BP-1267 |
| Gluconobacter oxydans E-1 | FERM-P Nr. 8353, | FERM BP-1265 |
| Gluconobacter oxydans H-2 | FERM-P Nr. 8354, | FERM BP-1266 |
| Gluconobacter oxydans L-8 | FERM-P Nr. 8355, | FERM BP-1268 |

Die Mikroorganismen können in wässrigem Medium, das die geeigneten Nährstoffe enthält, unter aeroben Bedingungen kultiviert werden. Die Kultivierung kann bei einem pH-Wert von 4,0 bis ca. 8,0, vorzugsweise 4,5 bis 6,5, durchgeführt werden. Die Kultivierungszeit hängt von der Natur des verwendeten Mikroorganismus und vom verwendeten Nährstoff ab, sie beträgt vorzugsweise ca. 10 bis 100 Stunden. Der bevorzugte Temperaturbereich zur Durchführung der Kultivierung beträgt 10-40°C, insbesondere 25-35°C.

Das Kulturmedium enthält normalerweise folgende Nährstoffe: assimilierbare Kohlenstoffquellen, wie Glycerin, D-Mannit, D-Sorbit, Erythrit, Ribit, Xylit, Arabit, Inosit, Dulcit, D-Ribose, D-Fructose, D-Frucose, D-Glucose, Gluconat, L-Sorbose, Maltose und Sucrose, vorzugsweise L-Sorbose, D-Sorbit oder Glycerin; abbaubare Stickstoffquellen, wie organische Substanzen, z.B. Pepton, Hefeextrakt, Sojabohnenmehl und Maisquellwasser; und anorganische Substanzen, z.B. Ammoniumsulfat, Ammoniumchlorid und Kaliumnitrit; Vitamine und Spurenelemente.

Im folgenden wird eine Ausführungsform für die Isolierung von L-Sorbose-Dehydrogenase aus den Mikroorganismen nach der Kultivierung und die Reinigung der L-Sorbose-Dehydrogenase beschrieben:

(1) Die Zellen werden aus der Fermentationsbrühe durch Zentrifugation geerntet.

(2) Die geernteten Zellen werden mit Wasser, physiologischer Kochsalzlösung oder einer Pufferlösung mit einem geeigneten pH-Wert gewaschen.

(3) Die gewaschenen Zellen werden in der Pufferlösung suspendiert und durch einen Homogenisator, ein Ultraschallgerät oder durch Behandlung mit Lysozym und dergleichen zerstört.

(4) Die L-Sorbose-Dehydrogenase wird aus dem zellfreien Extrakt des zerstörten Zellmaterials isoliert, vorzugsweise aus der Membranfraktion des Zellmaterials, und gereinigt.

Die erfindungsgemässe L-Sorbose-Dehydrogenase eignet sich als Katalysator zur Herstellung von L-Sorboson aus L-Sorbose. Die Reaktion wird zweckmässigerweise bei pH-Werten von ca. 5,0 bis 10,0 in Gegenwart eines Elektronenakzeptors, z.B. DCIP, Phenazinmethosulfat, Wurster's Blue, Ferricyanid, Coenzym Q, Cytochrom C und dergleichen, in einer Lösung, wie Phosphatpuffer, Tris-HCl-Puffer und dergleichen, durchgeführt. Ein bevorzugter Temperaturbereich zur Durchführung der Reaktion ist derjenige von ca. 20°C bis ca. 60°C. Die besten Ergebnisse werden bei pH-Werten im Bereich ca. 6,5-8,0 und bei 50°C erzielt. Die Konzentration von L-Sorbose in einer Lösung hängt auch noch von den weiteren Reaktionsbedingungen ab, liegt jedoch vorzugsweise zwischen 10 und 100 g/l, insbesondere zwischen 30 und 40 g/l.

Das Enzym kann auch im immobilisierten Zustand auf einem geeigneten Träger verwendet werden. Das Enzym kann auf irgendeine gängige Weise immobilisiert werden. Beispielsweise kann das Enzym unmittelbar an eine Membran oder an ein Harz, welche(s) funktionelle Gruppen aufweist, gebunden werden, oder es kann durch als Brücken dienenden Verbindungen, die bifunktionelle Gruppen aufweisen, z.B. Glutaraldehyd, an das Harz gebunden werden.

Die nachfolgenden Beispiele veranschaulichen die vorliegende Erfindung.

## Beispiel 1

### Herstellung von L-Sorbose-Dehydrogenase

(1) Kultivierung von Gluconobacter oxydans UV-10 (FERM-P No. 8422)

Die Agarschrägkultur von Gluconobacter oxydans UV-10 (FERM-P Nr. 8422) wurde in 5 ml des Medium Nr. 3B in einem Reagenzglas inokuliert und bei 27°C innert 3 Tagen in einer Reagenzglas-Schüttelvorrichtung kultiviert. Das Medium enthielt 7,0% L-Sorbose, 0,05% Glycerin, 1,5% Hefeextrakt (Oriental Co.), 0,25% Magnesiumsulfat-heptahydrat und 1,0% Calciumcarbonat. 1 ml dieser Kultur wurde in 50 ml des gleichen Mediums in einen 500 ml-Erlenmeyer-Kolben übertragen und bei 27°C innert 3 Tagen in einer Schüttelvorrichtung (180 Upm) kultiviert. 800 ml der so hergestellten Kultur wurden als Inoculum für einen 30 l-Fermenter verwendet, der 20 l des Mediums Nr. 3B enthielt. Der Fermenter wurde bei 30°C, 400 Upm sowie 1 Vvm (Volumen Luft/Volumen Medium/Minute) für Belüftung verwendet. Nach 40 Stunden Fermentation wurden die Zellen geerntet. Die Brühe wurde bei 1500 Upm (365 $\times$ g) 10 Minuten zentrifugiert, um das Calciumcarbonat abzutrennen, anschliessend bei 8000 Upm (10000 $\times$ g), um die Zellen zu sedimentieren. Der Zellkuchen wurde mit 0,85% wässriger Natriumchloridlösung einmal gewaschen. Aus 20 l Brühe wurden 374 g (Nettogewicht) Zellen erhalten.

(2) Herstellung der Membranfraktion

Die Zellen von Gluconobacter oxydans (77.5 g) der vorhergehenden Stufe (1) wurden zweimal mit physiologischer Kochsalzlösung gewaschen. Die gewaschenen Zellen wurden in 230 ml des Puffers suspendiert und mittels eines Dyno Mühle-Homogenisators (A.Willy, Bachofen Co., Basel) in Gegenwart von Glaskügelchen (0,1 mm ø) bei 2000 Upm 4 Minuten zerstört. Zelltrümmer wurden durch 10-minütige Zentrifugation bei 4000 Upm (1800 $\times$ g) entfernt, und anschliessend wurde der zellfreie Extrakt bei 40000 Upm (80000 $\times$ g) 1 Stunde zentrifugiert. Der resultierende Niederschlag wurde als Membranfraktion (42 g) abgetrennt.

Die Membranfraktion wurde in 600 ml des Puffers, enthaltend 0,5% Tween 80 und 0,2M L-Sorbose suspendiert. Man rührte die Suspension 3 Stunden und zentrifugierte sie bei 40000 Upm (80000 $\times$ g) 1 Stunde. Anschliessend wurde die Restmembran in 600 ml des Puffers, enthaltend 0,3% Triton X-100 und 0,2M L-Sorbose suspendiert. Die Suspension wurde 3 Stunden gerührt und dann bei 40000 Upm (80000 $\times$ g) 1 Stunde zentrifugiert. Der resultierende Ueberstand enthielt die solubilisierte L-Sorbose-Dehydrogenase. Man dialysierte den Ueberstand innert 16 Stunden gegen zweimal 2 l des Puffers, enthaltend 0,3% Triton X-100 und 0,2M L-Sorbose.

(3) Diäthylaminoäthyl (im folgenden als DEAE bezeichnet) -Cellulosesäulenchromatographie

Das Dialysat (570 ml) der vorhergehenden Stufe wurde auf eine DEAE-Cellulosesäule (2.5 $\times$ 50 cm) aufgetragen, die mit dem Puffer, enthaltend 0,3% Triton X-100 und 0,2M L-Sorbose äquilibriert worden war. Die Säule wurde mit demselben Puffer gewaschen, und die Elution des Enzyms wurde mit einem linearen Gradienten von Natriumchlorid bis zu 0,5M in demselben Puffer durchgeführt.

(4) DEAE-Sepharose-Säulenchromatographie

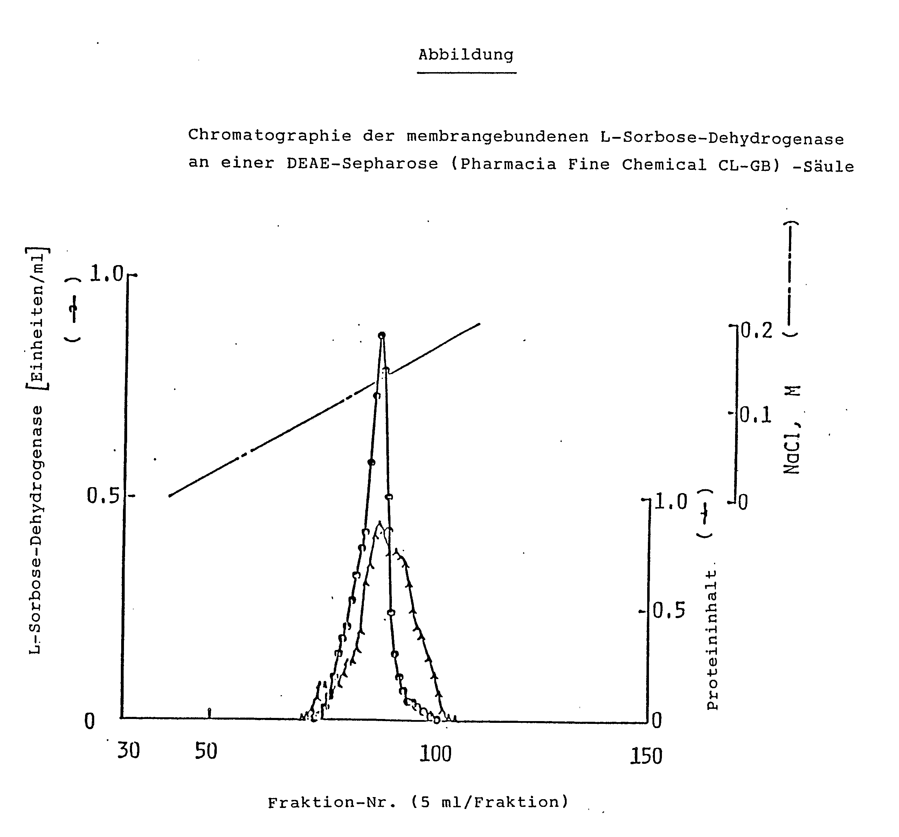

Die vereinigten Enzymfraktionen (100 ml) der vorhergehenden Stufe wurden gegen zweimal 1 l des Puffers, enthaltend 0,3% Triton X-100 und 0,2M L-Sorbose dialysiert, und auf eine DEAE-Sephrose-Säule (1,5 $\times$ 30 cm) aufgetragen, die mit demselben Puffer äquilibriert worden war. Nachdem die Säule mit demselben Puffer gewaschen worden war, wurde die L-Sorbose-Dehydrogenase mit einem linearen Gradienten von Natriumchlorid bus zu 0,2M, wie in der Abbildung gezeigt, eluiert. Die Fraktionen 87 bis 89 wurden vereinigt (15 ml) un in der nächsten Reiningungsstufe verwendet.

(5) Sephadex G-200-Säulenchromatographie

Eine Portion der Enzymfraktion (2 ml) wurde auf die Sephadex G-200-Säule (1,0 $\times$ 100 cm) aufgetragen, die mit demselben Puffer äquilibriert worden war, und eluiert. Die aktiven Fraktionen wurden vereinigt und bei -80°C gelagert. Die Reinigungsstufen des Enzyms sind in der Tabelle 8 zusammengefasst.

Tabelle 8

| Stufe | Total-protein (mg) | Total-aktivität (Einheiten) | spezifische Aktivität (Einheiten/mg) | Ausbeute (%) |
|---|---|---|---|---|
| zellfreier Extrakt | 7152 | 125,9 | 0,0176 | 100 |
| Membranfraktion | 2565 | 115,1 | 0,0449 | 91,4 |
| solubilisierte Membranfraktion | 504,5 | 78,04 | 0,155 | 62,0 |
| DEAE-Cellulose | 120,0 | 59,75 | 0,498 | 47,5 |
| DEAE-Sepharose | 38,35 | 29,44 | 0,768 | 23,4 |
| Sephadex G-200* | 0,6 | 0,538 | 0,897 | – |

* Eine einzige Portion der Fraktion der DEAE-Sepharose-Säulenchromatographie wurde für diese Stufe verwendet.

(6) Reinheit des isolierten Enzyms

Zur Bestimmung der Reinheit des isolierten Enzyms wurde eine Elektrophorese mit Polyacrylamidgel (Trenngel; 5% Acrylamid; 20 mA bei 4°C, 8 Stunden) durchgeführt. Das Enzym zeigte eine einzelne Bande, und es wurde durch 30-minütiges Anfärben mit der Lösung von 0,05M Phosphatpuffer (pH-Wert 7,0) enthaltend 50mM L-Sorbose, 40 μg/ml Nitroblautetrazolium und 140 μg/ml Phenazinmethosulfat bestätigt, dass dieses Protein in der Tat Enzymaktivität aufwies.

Die Elektrophorese mit Natriumdodecylsulfat (SDS)-Polyacrylamidgel (Trenngel; 15% Acrylamid; 20 mA bei Raumtemperatur, 3 Stunden) diente zur Bestimmung des Reinheitgrads und des Molekulargewichts des Enzyms. Das Enzym führte zu einer einzelnen Bande mit einem Molekulargewicht von 58.000 ± 5.000. Als Molekulargewichtstandard wurden Phosphorylase B (MW 92.500), Rinderserumalbumin (MW 66.200), Ovalbumin (MW 45.000), Carbonsäureanhydrase (MW 31.000), Trypsin-Inhibitor der Sojabohne (MW 21.500) und Lysozym (MW 14.400) verwendet.

(7) Identifikation des Reaktionsproduktes

Das Reaktionsgemisch enthaltend 0,2 ml des gereinigten Enzyms, 0,05 ml 0,5M Kaliumphosphatpuffer (pH-Wert 7,0), 0,05 ml 1M L-Sorbose, 0,02 ml 0,2M Phenazinmethosulfat und Wasser in einem Endvolumen von 0,5 ml wurde 30 Minuten bei 45°C inkubiert. Das Reaktionsprodukt wurde durch Dünnschichtchromatographie und Hochdruckflüssigkeitschromatographie analysiert. Das Produkt wurde durch Vergleich mit einer authentischen Probe als L-Sorboson identifiziert.

Beispiel 2

Nach der in Beispiel 1 beschriebenen Methode wurde L-Sorbose-Dehydrogenase aus den Stämmen Gluconobacter oxydans E-1 (FERM-P Nr. 8353), H-2 (FERM-P Nr. 8354) und L-8 (FERM-P Nr. 8355) isoliert und charakterisiert. Die aus diesen Stämmen gewonnenen Enzyme wiesen dieselben Eigenschaften wie diejenigen des Enzyms aus Gluconobacter oxydans UV-10 (FERM-P Nr. 8422) auf.

Beispiel 3

Das Reaktionsgemisch enthaltend 100 ml zellfreien Extrakt von Gluconobacter oxydans UV-10, FERM-P Nr. 8422 (Total-Enzymaktivität 123 Einheiten), hergestellt nach den oben beschriebenen Stufen (1) und (2) des Beispiels 1, 50 ml 0,5M Kaliumphosphatpuffer (pH-Wert 6,0), 50 ml 1M L-Sorbose-Lösung, 10 ml 0,2M Phenazinmethosulfat-Lösung und 290 ml Wasser wurde bei 30°C unter leichtem Schütteln inkubiert. Es bildete sich L-Sorboson und zwar 142 mg/Stunde.

Beispiel 4

Das Reaktionsgemisch enthaltend 400 ml gereinigte Membran-gebundene L-Sorbose-Dehydrogenase (Totalaktivität, 120 Einheiten), hergestellt nach den oben beschriebenen Stufen (1) bis (5) des Beispiels 1, 50 ml 0,5M Kaliumphosphatpuffer (pH-Wert 6,0), 50 ml 1M L-Sorbose-Lösung und 10 ml 0,2M Phenazinmethosulfat-Lösung wurde bei 30°C unter leichtem Schütteln inkubiert. Es bildeten sich L-Sorboson, und zwar 224 mg/Stunde.

**Ansprüche**

1. Coenzym-unabhängige L-Sorbose-Dehydrogenase, die die Oxidation von L-Sorbose zu L-Sorboson katalysiert, mit hoher Substratspezifität, gekennzeichnet durch die folgenden physiko-chemischen Eigenschaften:

      a) Optimaler pH-Wert: ca. 6,5-80

      b) Optimale Temperatur: 45-55°C

c) Molekulargewicht: bestehend aus identischen Untereinheiten mit einem Molekulargewicht von ca. 58.000 ± 5.000, gemessen mit Natriumdodecylsulfat-Polyacrylamidgelelektrophorese

d) Stabilisierung: durch L-Sorbose

e) Hemmung: durch $Cu^{2+}$ und Monojodacetat.

2. Verfahren zur Herstellung der neuen Coenzym-unabhängigen L-Sorbose-Dehydrogenase, dadurch gekennzeichnet, dass man einen zur Gattung Gluconobacter gehörenden Mikroorganismus oder eine Mutante davon, die zur Herstellung der neuen L-Sorbose-Dehydrogenase befähigt sind, kultiviert, das Zellmaterial zertrümmert, die L-Sorbose-Dehydrogenase aus dem zellfreiem Extrakt des zertrümmerten Zellmaterials, vorzugsweise aus der Membranfraktion des Zellmaterials, isoliert und die L-Sorbose-Dehydrogenase reinigt.

3. Verfahren zur Herstellung der neuen Coenzym-unabhängigen L-Sorbose-Dehydrogenase nach Anspruch 2, dadurch gekennzeichent, dass der Mikroorganismus zur Gattung Gluconobacter oxydans gehört.

4. Verfahren zur Herstellung der neuen Coenzym-unabhängigen L-Sorbose-Dehydrogenase nach Anspruch 3, dadurch gekennzeichnet, dass der Mikroorganismus aus der Gruppe Gluconobacter oxydans UV-10 (FERM-P Nr. 8422), Gluconobacter oxydans E-1 (FERM-P Nr. 8353), Gluconobacter oxydans H-2 (FERM-P Nr. 8354) und Gluconobacter oxydans L-8 (FERM-P Nr. 8355) ausgewählt wird.

5. Verfahren zur Oxidation von L-Sorbose zu L-Sorboson, dadurch gekennzeichnet, dass diese Oxidation durch die in Anspruch 1 angegebene L-Sorbose-Dehydrogenase katalysiert wird.

Chromatographie der membrangebundenen L-Sorbose-Dehydrogenase
an einer DEAE-Sepharose (Pharmacia Fine Chemical CL-GB) -Säule

Fraktion-Nr. (5 ml/Fraktion)